# FASCICULE DE BREVET EUROPEEN

(11) **EP 0 556 124 B1**
(45) Date de publication et mention de la délivrance du brevet: **05.06.1996**
(21) Numéro de dépôt: 93400349.2
(22) Date de dépôt: 11.02.1993
(51) Int. Cl.: C07K 14/575, A61K 38/22

(54) **Nouveaux dérivés peptidiques, procédé de préparation et application à titre de médicaments de ces nouveaux dérivés**
Neue Peptidderivate, Verfahren zu deren Herstellung und deren Anwendung als Heilmittel
New peptide derivatives, process to prepare them and their use as medicaments

(30) Priorité: 12.02.1992 FR 9201557
(43) Date de publication de la demande: 18.08.1993
(73) Titulaire: ROUSSEL UCLAF, F-93230 Romainville (FR)
(72) Inventeur: Hamon, Gilles, F-93340 Le Raincy (FR); Mahe, Eve, 825, Avenue du Comté de Nice, F-34080 Montpellier (FR); Le-Nguyen, Dung, F-34080 Montpellier (FR)
(74) Mandataire: Vieillefosse, Jean-Claude

(56) Documents cités:
- JOURNAL OF CARDIOVASCULAR PHARMACOLOGY vol. 17, no. SUP7, 1991, NEW YORK, NY, USA pages S5 - S9 T X WATANABE ET AL. 'the biological activity of endothelin-1 analogues in three different assay systems'
- CA 115, abrégé 107106

## Description

La présente demande concerne de nouveaux dérivés peptidiques, le procédé de préparation et l'application à titre de médicament de ces nouveaux dérivés.

On sait que le " V.I.C. " (Vasoactive Intestinal Contractor) est un puissant vasoconstricteur cloné et sequencé à partir du génome de souris et présent dans l'intestin de cette espèce. Ce peptide qui a été isolé récemment comporte 21 amino acides, présente 2 ponts disulfures et a pour formule : 
Cys Ser Cys Asn Ser Trp Leu Asp Lys Glu
1               5                   10
Cys Val Tyr Phe Cys His Leu Asp Ile Ile Trp
                15                  20
(Differential activities of two distinct endothelin family peptides on ileum and coronary artery: ISHIDA N., TSUJIOKA K., TOMOI, M.,SAIDA K. et MITSUI,Y.FEBS Letter,(1989) 247 pp. 337-340). Ce peptide est désigné dans ce qui suit sous le nom de VIC.

La nomenclature utilisée est celle de la commission IUPAC-IUB (1984) European J. Biochem 183, 9-37.

Ce peptide qui renferme 21 résidus d'amino-acides et 2 ponts disulfures possède une structure assez proche de celle de l'endothéline; il est capable, après liaison aux récepteurs de l'endothéline, de contracter à de très faibles doses les cellules des muscles lisses (artères et veines) de différentes espèces de mammifères (homme, chien, chat, cochon, cobaye, rat, lapin..). En essayant de raccourcir la chaine et de remplacer certains restes d'amino-acides du V.I.C. par d'autres amino-acides, la demanderesse a préparé par synthèse de nouveaux dérivés peptidiques dans lesquels l'activité vasoconstrictrice du V.I.C. a pratiquement disparu et qui présente de plus, la propriété de bloquer et de se fixer sur les récepteurs naturels de l'endothéline. Les produits présentent ainsi une activité qui s'opposera à toute action d'une libération ultérieure de l'endothéline.
Ces produits présentent en outre, l'intérêt de pouvoir être préparés par synthèse totale en grandes quantités.

La présente demande a ainsi pour objet de l'un des dérivés peptidiques suivants : 
Cys Ser Cys Phe Ser Ser Leu Asp Lys Glu Cys Val Tyr Phe Cys
1               5                   10                  15
His Leu Asp Ile Ile Trp,
20

Cys Ser Cys Gly Ser Trp Leu Asp Lys Glu Cys Val Tyr Phe Cys
1               5                   10                  15
His Leu Asp Ile Ile Trp,
20

Cys Ser Cys Asn Ser Ser Leu Asp Lys Glu Cys Val Tyr Phe Cys
1               5                   10                  15
His Leu Asp Ile Ile Trp,
20

Cys Ser Cys Phe Ser Thr Leu Asp Lys Glu Cys Val Tyr Tyr Cys
1               5                   10                  15
His Leu Asp Ile Ile Trp,
20

Cys Ser Cys Asn Ser Tyr Leu Asp Lys Glu Cys Val Tyr Phe Cys
1               5                   10                  15
His Leu Asp Ile Ile Trp,
20

Cys Ser Cys Phe Ser Lys Leu Asp Lys Glu Cys Val Tyr Phe Cys
1               5                   10                  15
His Leu Asp Ile Ile Trp,
20
 
Cys Ser Cys Asn Ser Ser Leu Asp Lys Glu Cys Val
1               5                   10
Tyr Phe Cys His Leu Asp Ile Ile
            15                  20

Cys Ser Cys Asn Ser Trp Leu Asp Lys Glu Cys Val
1               5                   10
Tyr Phe Cys His Leu Asp Ile Ile
            15                  20

Cys Ser Cys Phe Ser Trp Leu Asp Lys Glu Cys Val
1               5                   10

Tyr Phe Cys His Leu Asp Ile Ile Trp
        15                  20

Cys Ser Cys Phe Ser Ser Leu Asp Glu Glu Cys Val
1               5                   10
Tyr Phe Cys His Leu Asp Ile Ile Trp
            15                  20

Cys Ser Cys Phe Ser Asp Leu Asp Lys Glu Cys Val
1               5                   10
Tyr Phe Cys His Leu Asp Ile Ile Trp
        15                  20

Cys Ser Cys Asn Ser Trp Leu Asp Lys Glu Cys Val
1               5                   10
Tyr Phe Cys His Asp Asp Ile Ile Trp
        15                  20

Cys Ser Ala Phe Ser Ser Leu Asp Lys Glu Ala Val
1               5                   10
Tyr Phe Cys His Leu Asp Ile Ile Trp
        15                  20

Cys Ser Cys Asn Ser Thr Leu Asp Lys Glu Cys Val
1               5                   10
 
Tyr Phe Cys His Leu Asp Ile Ile Trp
15 20

H Ser Cys Phe Ser Ser Leu Asp Lys Glu Ala Val
1 5 10
Tyr Phe Cys His Leu Asp Ile Ile Trp
15 20

H Ser Cys Asn Ser Trp Leu Asp Lys Glu Ala Val
1 5 10
Tyr Phe Cys His Leu Asp Ile Ile Trp
15 20

Cys Ser Ala Asn Ser Trp Leu Asp Lys Glu Ala Val
1 5 10
Tyr Phe Cys His Leu Asp Ile Ile Trp
15 20
et leurs dérivés présentant le cas échéant un ou deux ponts disulfures.

Parmi les nouveaux dérivés peptidiques tels que définis ci-dessus, on retient tout particulièrement le dérivé peptidique suivant : 
Cys Ser Cys Phe Ser Ser Leu Asp Lys Glu Cys Val Tyr Phe Cys
1               5                   10                  15
His Leu Asp Ile Ile Trp,
                20
et ses dérivés présentant un ou deux ponts disulfures.

L'invention a également pour objet, un procédé de préparation des nouveaux dérivés peptidiques tels que définis ci-dessus, caractérisé en ce que l'on effectue une synthèse en phase solide en introduisant séquentiellement sur un support de type polystyrène réticulé les amino-acides dûment protégés, à l'aide d'un agent de couplage, déprotège les amino-acides, et libéré de la résine la channe peptidique ainsi formée, puis cyclise le cas échéant, le ou les ponts disulfures pour obtenir le dérivé peptidique ainsi recherché.

Dans des conditions préférentielles de mise en oeuvre, le procédé ci-dessus décrit est caractérisé en ce que :
- le support de type polystyrène réticulé est une résine de type "Boc-X₂₁-CM" dans laquelle Boc est un groupement tert-butyloxycarbonyl, X₂₁ a la signification déja indiquée et CM désigne le support de polystyrène réticulé,
- l'agent de couplage est le (benzotriazol-1-yloxy) tris(diméthylamino) phosphonium hexafluorophosphate ou Bop,
- la libération du support de la chaîne peptidique ainsi que la déprotection des amino-acides est effectuée au moyen de l'acide fluorhydrique en opérant à basse température de préférence vers 0°C.

Les nouveaux dérivés peptidiques, objet de la présente invention présentent de très intéressantes propriétés pharmacologiques ; on note en particulier un remarquable effet inhibiteur contre l'hypertension induite par l'endothéline et un effet antiischémique, l'activité vasoconstrictrice du V.I.C. étant abolie.

Certaines de ces propriétés sont illustrées plus loin dans la partie expérimentale.

Ces propriétés justifient l'utilisation des nouveaux dérivés peptidiques ci-dessus à titre de médicaments.

La présente invention a ainsi également pour objet, l'application à titre de médicaments des nouveaux dérivés peptidiques définis ci-dessus.

Parmi les médicaments préférés de l'invention, on retient tout particulièrement ceux constitués par l'un des dérivés peptidiques suivants : 
Cys Ser Cys Phe Ser Ser Leu Asp Lys Glu Cys Val Tyr Phe Cys
1               5                   10                  15
His Leu Asp Ile Ile Trp ,
                20

Cys Ser Cys Gly Ser Trp Leu Asp Lys Glu Cys Val Tyr Phe Cys
1               5                   10                  15
His Leu Asp Ile Ile Trp ,
                20

Cys Ser Cys Asn Ser Ser Leu Asp Lys Glu Cys Val Tyr Phe Cys
1               5                   10                  15
His Leu Asp Ile Ile Trp ,
                20

Cys Ser Cys Phe Ser Thr Leu Asp Lys Glu Cys Val Tyr Tyr Cys
1               5                   10                  15
His Leu Asp Ile Ile Trp ,
                20

Cys Ser Cys Asn Ser Tyr Leu Asp Lys Glu Cys Val Tyr Phe Cys
1               5                   10                  15
His Leu Asp Ile Ile Trp ,
                20
 
Cys Ser Cys Phe Ser Lys Leu Asp Lys Glu Cys Val Tyr Phe Cys
1               5                   10                  15
His Leu Asp Ile Ile Trp ,
                20
et leurs dérivés présentant le cas échéant un ou deux ponts disulfures.

Parmi les médicaments préférés de l'invention on retient tout particulièrement le dérivé peptidique suivant: 
Cys Ser Cys Phe Ser Ser Leu Asp Lys Glu Cys Val Tyr Phe Cys
1               5                   10                  15
His Leu Asp Ile Ile Trp ,
                20
et ses dérivés présentant un ou deux ponts disulfures.

Ces médicaments trouvent, par exemple, leur emploi dans le traitement de tous les spasmes vasculaires, dans le traitement des post-hémorragies cérébrales, dans le traitement des spasmes coronariens, des spasmes vasculaires périphériques ainsi que dans le traitement des insuffisances rénales. Ces médicaments peuvent également être utilisés dans le traitement de l'infarctus du myocarde, dans la prévention des resténoses post-angioplastie, le traitement de l'athérosclérose, de certaines formes d'hypertension, ainsi que dans le traitement de l'asthme.

La dose usuelle, variable selon le produit utilisé, le sujet traité et l'affection en cause, peut être par exemple de 1 à 300 mg par jour par voie intraveineuse chez l'homme.

L'invention a également pour objet les compositions pharmaceutiques qui renferment au moins un dérivé précité à titre de principe actif.

A titre de principe actif, les dérivés peptidiques ci-dessus, peuvent être des compositions pharmaceutiques destinées à la voie digestive ou parentérale.

Ces compositions pharmaceutiques peuvent être, par exemple, solides ou liquides et se présenter sous les formes pharmaceutiques couramment utilisées en médecine humaine, comme par exemple, les comprimés simples ou dragéifiés, les gélules, les capsules, les granulés, les suppositoires, les préparations injectables, les aérosols ; elles sont préparées selon les méthodes usuelles. Le ou les principes actifs peuvent être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

Il va être donné maintenant à titre non limitatif, des exemples de mise en oeuvre de l'invention.

### EXEMPLE 1 :

Cys Ser Cys Asn Ser Ser Leu Asp Lys Glu Cys Val
1               5                   10
Tyr Phe Cys His Leu Asp Ile Ile
        15                  20

(dérivé de formule (I) (X₁ = Cys X₃ = Cys X₄ = Asn X₆ = Ser X₉ = Lys X₁₁ = Cys X₁₄ = Phe X₁₇ = Leu X₂₁ = H Avec 2 ponts disulfures entre les cystéines

### STADE A : Assemblage de la chaîne peptidique

On utilise un support de type "Boc-Trp-CM-support" dans laquelle Boc est un groupement tert-butyloxycarbonyl, Trp représente un groupement tryptophyl et CM-support désigne le support de polystyrène réticulé.

Le support renfermant 0,6 mmol Trp/g a été préparé par la méthode au fluorure de potassium selon la technique décrite par Horiki K., Igano K. & Inouye K.(1978) Chemistry Lett., 165-168.
Tous les aminoacides sont M-protégés par le groupement tert-tert-butyloxycarbonyl, les groupements protecteurs des chaînes latérales ont été les suivants :
- p-méthyl benzyl pour la cystéine,
- ester de cyclohexyl pour l'acide aspartique et l'acide glutamique,
- benzyl pour la sérine,
- o-chlorobenzyloxycarbonyl pour la lysine,
- 2,6-dichlorobenzyl pour la tyrosine,
- Nim-tertiobutyloxycarbonyl pour l'histidine,
- le tryptophane n'est pas protégé.

Les réactions de couplage ont été effectuées en utilisant le Bop ou (benzotriazol-1-yloxy tris (diméthylamino) phosphonium hexafluorophosphate.

Un cycle de couplage peut être résumé comme suit :

### Déprotection :

1 - Traitement avec une solution d'acide trifluoroacétique (50 %) dans le dichlorométhane contenant 3 % d'éthanedithiol pendant 1 minute.
2 - Vidange puis traitement avec une solution d'acide trifluoroacétique à 50 % dans le dichlorométhane contenant 3 % d'éthanedithiol pendant 30 minutes.
3 - Vidange puis lavage avec de l'isopropanol contenant 5 % d'éthanedithiol.
4 - Lavage au dichlorométhane, 2 fois.

### Couplage :

1 - Addition de Bop et de Boc-acide aminé.
2 - Addition de diisopropyléthylamine (6 équivalents) puis de solvant (dichlorométhane ou diméthylformamide) sous agitation.
3 - Après réaction négative à la ninhydrine lavage 2 fois au dichlorométhane.
Les tests de contrôle utilisant la ninhydrine ont été effectués suivant la méthode décrit par Kaiser, E., Colescott, R.L., Bossinger, C.D. & Cook, P.I. (1970) Anal. Biochem. 24, 595-598.
Avant le traitement par l'acide fluorhydrique le dernier groupement Boc est éliminé par l'acide trifluoroacétique.

Au départ de 2 g de résine " Boc-Trp-CM-résine" on a ainsi obtenu en opérant manuellement 5,25 g de complexe chaîne peptidique protégée-résine que l'on utilise directement au stade suivant.

### STADE B : Libération du complexe chaîne peptidique protégée-résine

On fait réagir 1,5 g de complexe chaîne peptidique protégée-résine pendant 60 minutes avec 15 ml d'acide fluorhydrique à 0°C en présence d'anisole (1 ml) et diméthylsulfide (0,5 ml). La résine est lavée à l'éther ; le peptide brut est extrait par une solution aqueuse d'acide acétique à 20 %. Après lyophilisation, on obtient 660 mg de produit brut non cyclisé que l'on utilise directement au stade suivant.

### STADE C : Cyclisation et purification

On dissout 500 mg du peptide brut obtenu ci-dessus, dans 500 ml d'eau, agite vigoureusement, ajoute de la diisopropyl éthylamine jusqu'à obtention de pH 8 (une goutte de mélange est recueillie toutes les heures et ajoutée à une goutte d'une solution contenant de l'acide dithiobis (2-nitrobenzoique) dans un tampon molaire de K₂HPO₄ (pH 8) pour suivre la réaction d'oxydation). Pendant toute la réaction on maintient le pH à 8 par addition de diisopropyléthylamine. La réaction est suivie par chromatographie liquide haute performance (HPLC).

Après 28 heures, on constate l'absence de coloration jaune dans le test à l'acide dithiobis (2-nitrobenzoique).

Le produit obtenu est purifié par chromatographie liquide haute performance (HPLC) sur colonne Whatman M20 ODS₃ et élué par un mélange eau-acétonitrile renfermant 0,1 % d'acide trifluoroacétique.

On lyophilise la fraction obtenue et recueille finalement 30 mg de produit attendu.

### EXEMPLES 2 à 16 :

En opérant de la même manière qu'à l'exemple 1 mais avec d'autres amino-acides, on a préparé les 15 autres microprotéines qui figurent ci-après.

### EXEMPLE 2 :

Cys Ser Cys Asn Ser Trp Leu Asp Lys Glu Cys Val
1               5                   10
Tyr Phe Cys His Leu Asp Ile Ile
        15                  20

(dérivé de formule (I) (X₁ = Cys X₃ = Cys X₄ = Asn X₆ = Trp X₉ = Lys X₁₁ = Cys X₁₄ = Phe X₁₇ = Leu X₂₁ = H Avec 2 ponts disulfures entre les cystéines 1-15 et 3-11.

### EXEMPLE 3 :

Cys Ser Cys Phe Ser Trp Leu Asp Lys Glu Cys Val
1               5                   10

Tyr Phe Cys His Leu Asp Ile Ile Trp
        15                  20

(dérivé de formule (I) (X₁ = Cys X₃ = Cys X₄ = Phe X₆ = Trp X₉ = Lys X₁₁ = Cys X₁₄ = Phe X₁₇ = Leu X₂₁ = Trp Avec 2 ponts disulfures entre les cystéines 1-15 et 3-11.

### EXEMPLE 4 :

Cys Ser Cys Phe Ser Ser Leu Asp Lys Glu Cys Val
1               5                   10
Tyr Phe Cys His Leu Asp Ile Ile Trp
        15                  20

(dérivé de formule (I) (X₁ = Cys X₃ = Cys X₄ = Phe X₆ = Ser X₉ = Lys X₁₁ = Cys X₁₄ = Phe X₁₇ = Leu X₂₁ = Trp Avec 2 ponts disulfures entre les cystéines 1-15 et 3-11.

### EXEMPLE 5 :

Cys Ser Cys Asn Ser Tyr Leu Asp Lys Glu Cys Val
1               5                   10
Tyr Phe Cys His Leu Asp Ile Ile Trp
    15                  20

(dérivé de formule (I) (X₁ = Cys X₃ = Cys X₄ = Asn X₆ = Tyr X₉ = Lys X₁₁ = Cys X₁₄ = Phe X₁₇ = Leu X₂₁ = Trp Avec 2 ponts disulfures entre les cystéines 1-15 et 3-11.

### EXEMPLE 6 :

Cys Ser Cys Phe Ser Ser Leu Asp Glu Glu Cys Val
1               5                   10
Tyr Phe Cys His Leu Asp Ile Ile Trp
    15                  20

(dérivé de formule (I) (X₁ = Cys X₃ = Cys X₄ = Phe X₆ = Ser X₉ = Glu X₁₁ = Cys X₁₄ = Phe X₁₇ = Leu X₂₁ = Trp Avec 2 ponts disulfures entre les cystéines 1-15 et 3-11.

### EXEMPLE 7 :

Cys Ser Cys Gly Ser Trp Leu Asp Lys Glu Cys Val
1               5                   10
Tyr Phe Cys His Leu Asp Ile Ile Trp
        15                  20

(dérivé de formule (I) (X₁ = Cys X₃ = Cys X₄ = Gly X₆ = Trp X₉ = Lys X₁₁ = Cys X₁₄ = Phe X₁₇ = Leu X₂₁ = Trp Avec 2 ponts disulfures entre les cystéines 1-15 et 3-11.

### EXEMPLE 8 :

Cys Ser Cys Asn Ser Ser Leu Asp Lys Glu Cys Val
1               5                   10
Tyr Phe Cys His Leu Asp Ile Ile Trp
        15                  20

(dérivé de formule (I) (X₁ = Cys X₃ = Cys X₄ = Asn X₆ = Ser X₉ = Lys X₁₁ = Cys X₁₄ = Phe X₁₇ = Leu X₂₁ = Trp Avec 2 ponts disulfures entre les cystéines 1-15 et 3-11.

### EXEMPLE 9 :

Cys Ser Cys Phe Ser Asp Leu Asp Lys Glu Cys Val
1               5                   10
Tyr Phe Cys His Leu Asp Ile Ile Trp
        15                  20

(dérivé de formule (I) (X₁ = Cys X₃ = Cys X₄ = Ser X₆ = Leu X₉ = Lys X₁₁ = Cys X₁₄ = Phe X₁₇ = Leu X₂₁ = Trp Avec 2 ponts disulfures entre les cystéines 1-15 et 3-11.

### EXEMPLE 10 :

Cys Ser Cys Phe Ser Lys Leu Asp Lys Glu Cys Val
1               5                   10
Tyr Phe Cys His Leu Asp Ile Ile Trp
        15                  20

(dérivé de formule (I) (X₁ = Cys X₃ = Cys X₄ = Phe X₆ = Lys X₉ = Lys X₁₁ = Cys X₁₄ = Phe X₁₇ = Leu X₂₁ = Trp Avec 2 ponts disulfures entre les cystéines 1-15 et 3-11.

### EXEMPLE 11 :

Cys Ser Cys Phe Ser Thr Leu Asp Lys Glu Cys Val
1               5                   10
Tyr Tyr Cys His Leu Asp Ile Ile Trp
        15                  20

(dérivé de formule (I) (X₁ = Cys X₃ = Cys X₄ = Phe X₆ = Thr X₉ = Lys X₁₁ = Cys X₁₄ = Tyr X₁₇ = Leu X₂₁ = Trp Avec 2 ponts disulfures entre les cystéines 1-15 et 3-11.

### EXEMPLE 12 :

Cys Ser Cys Asn Ser Trp Leu Asp Lys Glu Cys Val
1               5                   10
Tyr Phe Cys His Asp Asp Ile Ile Trp
    15                  20

(dérivé de formule (I) (X₁ = Cys X₃ = Cys X₄ = Asn X₆ = Trp X₉ = Lys X₁₁ = Cys X₁₄ = Phe X₁₇ = Leu X₂₁ = Trp Avec 2 ponts disulfures entre les cystéines 1-15 et 3-11.

### EXEMPLE 13 :

Cys Ser Ala Phe Ser Ser Leu Asp Lys Glu Ala Val
1               5                   10
Tyr Phe Cys His Leu Asp Ile Ile Trp
    15                  20

(dérivé de formule (I) (X₁ = Cys X₃ = Ala X₄ = Phe X₆ = Ser X₉ = Lys X₁₁ = Ala X₁₄ = Phe X₁₇ = Leu X₂₁ = Trp Avec 1 pont disulfure entre les cystéines 1 et 15.

### EXEMPLE 14 :

Cys Ser Cys Asn Ser Thr Leu Asp Lys Glu Cys Val
1               5                   10
Tyr Phe Cys His Leu Asp Ile Ile Trp
        15                  20

(dérivé de formule (I) (X₁ = Cys X₃ = Cys X₄ = Asn X₆ = Threo X₉ = Lys X₁₁ = Cys X₁₄ = Phe X₁₇ = Leu X₂₁ = Trp Avec 2 ponts disulfures entre les cystéines 1-15 et 3-11.

### EXEMPLE 15 :

H Ser Cys Phe Ser Ser Leu Asp Lys Glu Ala Val
1               5                   10
Tyr Phe Cys His Leu Asp Ile Ile Trp
        15                  20

(dérivé de formule (I) (X₁ = H X₃ = Cys X₄ = Phe X₆ = Ser X₉ = Lys X₁₁ = Ala X₁₄ = Phe X₁₇ = Leu X₂₁ = Trp Avec un pont disulfure entre les cystéines 2 et 14.

### EXEMPLE 16 :

H Ser Cys Asn Ser Trp Leu Asp Lys Glu Ala Val
1 5 10
Tyr Phe Cys His Leu Asp Ile Ile Trp
15 20

(dérivé de formule (I) (X₁ = H X₃ = Cys X₄ = Asn X₆ = Trp X₉ = Lys X₁₁ = Ala X₁₄ = Phe X₁₇ = Leu X₂₁ = Trp Avec 1 pont disulfure entre les cystéines 2 et 14.

### EXEMPLE 17 :

Cys Ser Ala Asn Ser Trp Leu Asp Lys Glu Ala Val
1               5                   10
Tyr Phe Cys His Leu Asp Ile Ile Trp
        15                  20

(dérivé de formule (I) (X₁ = Cys X₃ = Ala X₄ = Asn X₆ = Trp X₉ = Lys X₁₁ = Ala X₁₄ = Phe X₁₇ = Leu X₂₁ = Trp Avec un pont disulfure entre les cystéines 1-15.

Chaque produit a été séquencé et sa masse a été vérifiée en spectrométrie de masse.

### EXEMPLE 18 :

On a préparé un soluté injectable répondant à la formulation suivante :

| | |
|---|---|
| - Produit de l'exemple 1 | 1 mg |
| - excipient aqueux stérile | 2 ml |

### EXEMPLE 19 :

On a préparé des comprimés répondant à la formule suivante :

| | |
|---|---|
| - Produit de l'exemple 1 | 2 mg |
| - excipient q.s.p. un comprimé terminé à (composition de l'excipient : lactose, amidon, talc, stéarate de magnésium). | 150 mg |

### ETUDE DE L'ACTIVITE SUR RECEPTEUR DE L'ENDOTHELINE

On effectue une préparation membranaire à partir de cortex postérieur plus cervelet de rat. Le tissu est broyé au POLYTRON dans un tampon Tris 50 mM pH = 7,4.
Après 30 minutes à 25 °C (B.M.) l'homogénat est centrifugé à 30000 g pendant 15 minutes (2 centrifugations avec reprise intermediaire dans le tampon Tris pH. 7,4).
Les culots sont remis en suspension dans un tampon d'incubation (Tris 25 mM, pepstatine A 5 microg/ml, aprotinine 3 microg/ml, PMSF 0,1 mM, EDTA 3mM, EGTA 1mM pH 7,4).
On répartit des aliquotes de 2 ml dans des tubes à hémolyse et ajoute de la I¹²⁵ Endothéline (environ 50000 dpm/tube) et le produit à étudier. (Le produit est d'abord testé à 3*10⁻⁵ M en triple). Lorsque le produit testé déplace de plus de 50 % la radioactivité liée spécifiquement au récepteur, il est testé à nouveau selon une gamme de 7 concentrations afin de déterminer la concentration qui inhibe de 50 % la radioactivité liée spécifiquement au récepteur. On détermine ainsi la concentration inhibitrice 50 %.
La liaison non spécifique est déterminée par addition d'endothéline à 10⁻⁶ M (en triple). On incube à 25 °C pendant 60 minutes, remet au bain-marie à 0 °C, pendant 5 minutes, filtre sous pression réduite, rince au tampon Tris pH 7,4 et compte la radioactivité en présence du scintillant Triton.
Le résultat est exprimé directement en concentration inhibitrice 50 % (CI50), c'est-à-dire en concentration de produit étudié exprimée en nM, nécessaire pour déplacer 50 % de la radioactivité spécifique fixée sur le récepteur étudié.

### Résultat :

Les CI50 trouvées pour les produits des exemples 1 à 17 sont données dans le tableau I ci-après, en nanomoles.

**TABLEAU I**

| EXEMPLES | CI50 (nM) |
|---|---|
| 2 | 938 |
| 3 | 2,6 |
| 4 | 3,0 |
| 5 | 1,2 |
| 6 | 2,9 |
| 7 | 0,9 |
| 8 | 0,9 |
| 9 | 2,4 |
| 10 | 1,8 |
| 11 | 1,0 |
| 12 | 6,8 |
| 13 | 1,4 |
| 14 | 2,2 |
| 15 | 17 |
| 16 | 7,2 |
| 17 | 1,9 |

### ETUDE DE L'ACTIVITE VASOCONSTRICTRICE ET DE L'ANTAGONISME DES EFFETS DE L'ENDOTHELINE CHEZ LE RAT DEMEDULLE

Des rats mâles Sprague-Dawley sont anesthésiés au nembutal (60 mg/kg) injecté par voie intrapéritonéale. Les animaux sont ensuite démédullés, mis sous assistance respiratoire (1 ml/100 g - 50 insuflations par minute), puis les nerfs vagues sont sectionnés. Une artère carotide est cathéterisée pour enregistrer la pression artérielle et les produits à étudier sont injectés dans la veine pudendale.

### . Etude de l'activité vasoconstrictrice

Les animaux reçoivent l'endothéline 1 ou le composé à tester en injections cumulées par voie veineuse, les administrations étant espacées de 2 minutes.

L'augmentation de pression artérielle moyenne (PAm) induite par chaque composé est mesurée et exprimée en pourcentage de variation par rapport à la pression initiale.

### . Etude de l'activité antagoniste à l'endothéline

Dix minutes après l'injection intraveineuse du solvant ou des composés à tester, les animaux reçoivent des administrations d'endothéline 1 de façon cumulative (1, 3, 10 et 30 µg/kg i.v.) chaque 2 minutes.

L'activité antagoniste est exprimée en pourcentage d'inhibition de la réponse pressive de l'endothéline administrée seule, aux doses de 3 et 10 µg/kg.

### Résultats :

L'activité vasoconstrictrice du composé de l'exemple 4 est donnée dans le tableau II ci-après :

**Tableau II**

| Exemple | Dose (µg/kg) | % d'augmentation de PAm |
|---|---|---|
| 4 | 1 | + 16% |

L'activité antagoniste du composé de l'exemple 4 est donnée dans le tableau III ci-après :

**Tableau III**

| Exemple | Dose (µg/kg) | Pourcentage d'inhibition des réponses à l'endothéline | |
|---|---|---|---|
| | | endothéline 3 µg | endothéline 10 µg |
| 4 | 1 | - 43 % | - 45 % |

## Revendications

1. Dérivés peptidiques, dont les noms suivent : 
Cys Ser Cys Phe Ser Ser Leu Asp Lys Glu Cys Val Tyr Phe Cys
1               5                   10                  15
His Leu Asp Ile Ile Trp,
                20

Cys Ser Cys Gly Ser Trp Leu Asp Lys Glu Cys Val Tyr Phe Cys
1               5                   10                  15
His Leu Asp Ile Ile Trp,
                20

Cys Ser Cys Asn Ser Ser Leu Asp Lys Glu Cys Val Tyr Phe Cys
1               5                   10                  15
His Leu Asp Ile Ile Trp,
                20

Cys Ser Cys Phe Ser Thr Leu Asp Lys Glu Cys Val Tyr Tyr Cys
1               5                   10                  15
His Leu Asp Ile Ile Trp,
                20

Cys Ser Cys Asn Ser Tyr Leu Asp Lys Glu Cys Val Tyr Phe Cys
1               5                   10                  15
His Leu Asp Ile Ile Trp,
                20

Cys Ser Cys Phe Ser Lys Leu Asp Lys Glu Cys Val Tyr Phe Cys
1               5                   10                  15
His Leu Asp Ile Ile Trp,
                20

Cys Ser Cys Asn Ser Ser Leu Asp Lys Glu Cys Val
1               5                   10
Tyr Phe Cys His Leu Asp Ile Ile
        15                  20

Cys Ser Cys Asn Ser Trp Leu Asp Lys Glu Cys Val
1               5                   10
 
Tyr Phe Cys His Leu Asp Ile Ile
15 20

Cys Ser Cys Phe Ser Trp Leu Asp Lys Glu Cys Val
1 5 10

Tyr Phe Cys His Leu Asp Ile Ile Trp
15 20

Cys Ser Cys Phe Ser Ser Leu Asp Glu Glu Cys Val
1 5 10
Tyr Phe Cys His Leu Asp Ile Ile Trp
15 20

Cys Ser Cys Phe Ser Asp Leu Asp Lys Glu Cys Val
1 5 10
Tyr Phe Cys His Leu Asp Ile Ile Trp
15 20

Cys Ser Cys Asn Ser Trp Leu Asp Lys Glu Cys Val
1 5 10
Tyr Phe Cys His Asp Asp Ile Ile Trp
15 20

Cys Ser Ala Phe Ser Ser Leu Asp Lys Glu Ala Val
1 5 10
Tyr Phe Cys His Leu Asp Ile Ile Trp
15 20

Cys Ser Cys Asn Ser Thr Leu Asp Lys Glu Cys Val
1 5 10
Tyr Phe Cys His Leu Asp Ile Ile Trp
15 20

H Ser Cys Phe Ser Ser Leu Asp Lys Glu Ala Val
1 5 10
Tyr Phe Cys His Leu Asp Ile Ile Trp
15 20
 
H Ser Cys Asn Ser Trp Leu Asp Lys Glu Ala Val
1               5                   10
Tyr Phe Cys His Leu Asp Ile Ile Trp
        15                  20

Cys Ser Ala Asn Ser Trp Leu Asp Lys Glu Ala Val
 1              5                   10
Tyr Phe Cys His Leu Asp Ile Ile Trp
        15                  20
 et leurs dérivés présentant le cas échéant un ou deux ponts disulfures.

2. Dérivé peptidique, dont le nom suit : 
Cys Ser Cys Phe Ser Ser Leu Asp Lys Glu Cys Val Tyr Phe Cys
1               5                   10                  15
His Leu Asp Ile Ile Trp,
                20
 et ses dérivés présentant un ou deux ponts disulfures.

3. Procédé de préparation des nouveaux dérivés peptidiques tels que définis à la revendication 1, caractérisé en ce que l'on effectue une synthèse en phase solide en introduisant séquentiellement sur un support de type polystyrène réticulé les amino-acides dûment protégés, à l'aide d'un agent de couplage, déprotège les amino-acides, et libère de la résine la chaîne peptidique ainsi formée, puis cyclise le cas échéant, le ou les ponts disulfures pour obtenir les dérivés peptidiques ainsi recherchés.

4. Procédé selon la revendication 3, caractérisé en ce que :
- le support de type polystyrène réticulé est une résine de type "Boc-X₂₁-CM" dans laquelle Boc est un groupement tert-butyloxycarbonyl, X₂₁ a la signification déja indiquée et CM désigne le support de polystyrène réticulé,
- l'agent de couplage est le (benzotriazol-1-yloxy) tris(diméthylamino) phosphonium hexafluorophosphate ou Bop,
- la libération du support de la chaîne peptidique ainsi que la déprotection des amino-acides est effectuée au moyen de l'acide fluorhydrique en opérant à basse température de préférence vers 0°C.

5. Médicaments, caractérisés en ce qu'ils sont constitués par las nouveaux dérivés peptidiques tels que définis à la revendication 1.

6. Compositions pharmaceutiques, caractérisées en ce qu'elles renferment à titre de principe actif, l'un au moins des médicaments tels que définis à l'une quelconque des revendications 5 ou 6.

## Claims

1. Peptide derivatives, the names of which follow: 
Cys Ser Cys Phe Ser Ser Leu Asp Lys Glu Cys Val
1               5                   10
Tyr Phe Cys His Leu Asp Ile Ile Trp
        15                  20

Cys Ser Cys Gly Ser Trp Leu Asp Lys Glu Cys Val
1               5                   10
Tyr Phe Cys His Leu Asp Ile Ile Trp
        15                  20

Cys Ser Cys Asn Ser Ser Leu Asp Lys Glu Cys Val
1               5                   10
Tyr Phe Cys His Leu Asp Ile Ile Trp
        15                  20

Cys Ser Cys Phe Ser Thr Leu Asp Lys Glu Cys Val
1               5                   10
Tyr Tyr Cys His Leu Asp Ile Ile Trp
        15                  20

Cys Ser Cys Asn Ser Trp Leu Asp Lys Glu Cys Val
1               5                   10
Tyr Phe Cys His Leu Asp Ile Ile Trp
        15                  20

Cys Ser Cys Phe Ser Lys Leu Asp Lys Glu Cys Val
1               5                   10
Tyr Phe Cys His Leu Asp Ile Ile Trp
        15                  20

Cys Ser Cys Asn Ser Ser Leu Asp Lys Glu Cys Val
1               5                   10
 
Tyr Phe Cys His Leu Asp Ile Ile
        15                  20

Cys Ser Cys Asn Ser Trp Leu Asp Lys Glu Cys Val
  1               5                  10
Tyr Phe Cys His Leu Asp Ile Ile
        15                  20

Cys Ser Cys Phe Ser Trp Leu Asp Lys Glu Cys Val
  1               5                  10

Tyr Phe Cys His Leu Asp Ile Ile Trp
        15                  20

Cys Ser Cys Phe Ser Ser Leu Asp Glu Glu Cys Val
  1               5                  10
Tyr Phe Cys His Leu Asp Ile Ile Trp
        15                  20

Cys Ser Cys Phe Ser Asp Leu Asp Lys Glu Cys Val
  1               5                  10
Tyr Phe Cys His Leu Asp Ile Ile Trp
        15                  20

Cys Ser Cys Asn Ser Trp Leu Asp Lys Glu Cys Val
  1               5                  10
Tyr Phe Cys His Asp Asp Ile Ile Trp
        15                  20

Cys Ser Ala Phe Ser Ser Leu Asp Lys Glu Ala Val
  1               5                  10
Tyr Phe Cys His Leu Asp Ile Ile Trp
        15                  20

Cys Ser Cys Asn Ser Thr Leu Asp Lys Glu Cys Val
  1               5                  10
Tyr Phe Cys His Leu Asp Ile Ile Trp
        15                  20
 
H Ser Cys Phe Ser Ser Leu Asp Lys Glu Ala Val
1               5                   10
Tyr Phe Cys His Leu Asp Ile Ile Trp
        15                  20

H Ser Cys Asn Ser Trp Leu Asp Lys Glu Ala Val
1               5                   10
Tyr Phe Cys His Leu Asp Ile Ile Trp
        15                  20

Cys Ser Ala Asn Ser Trp Leu Asp Lys Glu Ala Val
 1              5                   10
Tyr Phe Cys His Leu Asp Ile Ile Trp
        15                  20
 and their derivatives having if appropriate one or two disulphide bridges.

2. Peptide derivative, the name of which follows: 
Cys Ser Cys Phe Ser Ser Leu Asp Lys Glu Cys Val Tyr Phe Cys
1               5                   10                  15
His Leu Asp Ile Ile Trp,
                20
 and its derivatives having one or two disulphide bridges.

3. Preparation process for new peptide derivatives as defined in claim 1, characterized in that a solid phase synthesis is carried out by introducing sequentially the duly protected amino acids on a cross-linked polystyrene type support, using a conjugation agent, the amino acids are deprotected and the peptide chain thus formed is released from the resin, then, if appropriate, the disulphide bridge or bridges are cyclized in order to obtain the sought peptide derivatives.

4. Process according to claim 3, characterized in that:
- the cross-linked polystyrene type support is a resin of "Boc-X₂₁-CM" type in which Boc is a tert-butyloxycarbonyl group, X₂₁ has the meaning already indicated and CM designates the cross-linked polystyrene support,
- the conjugation agent is (benzotriazol-1-yloxy) tris(dimethylamino) phosphonium hexafluorophosphate or Bop,
- release of the peptide chain from the support as well as the deprotection of the amino acids is carried out using fluorohydric acid by operating at low temperature, preferably about 0°C.

5. Medicaments, characterized in that they are constituted by the new peptide derivatives as defined in claim 1.

6. Pharmaceutical compositions, characterized in that they contain as active ingredient, at least one of the medicaments as defined in any one of claims 5 or 6.

## Patentansprüche

1. Peptid-Derivate mit den folgenden Namen : 
Cys Ser Cys Phe Ser Ser Leu Asp Lys Glu Cys Val Tyr Phe Cys
1               5                   10                  15
His Leu Asp Ile Ile Trp,
                20

Cys Ser Cys Gly Ser Trp Leu Asp Lys Glu Cys Val Tyr Phe Cys
1               5                   10                  15
His Leu Asp Ile Ile Trp,
                20

Cys Ser Cys Asn Ser Ser Leu Asp Lys Glu Cys Val Tyr Phe Cys
1               5                   10                  15
His Leu Asp Ile Ile Trp,
                20

Cys Ser Cys Phe Ser Thr Leu Asp Lys Glu Cys Val Tyr Tyr Cys
1               5                   10                  15
His Leu Asp Ile Ile Trp,
                20

Cys Ser Cys Asn Ser Tyr Leu Asp Lys Glu Cys Val Tyr Phe Cys
1               5                   10                  15
His Leu Asp Ile Ile Trp,
                20

Cys Ser Cys Phe Ser Lys Leu Asp Lys Glu Cys Val Tyr Phe Cys
1               5                   10                  15
His Leu Asp Ile Ile Trp,
                20
 
Cys Ser Cys Asn Ser Ser Leu Asp Lys Glu Cys Val Tyr Phe Cys
1               5                   10                  15
His Leu Asp Ile Ile,
                20

Cys Ser Cys Asn Ser Trp Leu Asp Lys Glu Cys Val Tyr Phe Cys
1               5                   10                  15
His Leu Asp Ile Ile,
                20

Cys Ser Cys Phe Ser Trp Leu Asp Lys Glu Cys Val Tyr Phe Cys
1               5                   10                  15
His Leu Asp Ile Ile Trp,
                20

Cys Ser Cys Phe Ser Ser Leu Asp Glu Glu Cys Val Tyr Phe Cys
1               5                   10                  15
His Leu Asp Ile Ile Trp,
                20

Cys Ser Cys Phe Ser Asp Leu Asp Lys Glu Cys Val Tyr Phe Cys
1               5                   10                  15
His Leu Asp Ile Ile Trp,
                20

Cys Ser Cys Asn Ser Trp Leu Asp Lys Glu Cys Val Tyr Phe Cys
1               5                   10                  15
His Asp Asp Ile Ile Trp,
                20

Cys Ser Ala Phe Ser Ser Leu Asp Lys Glu Ala Val Tyr Phe Cys
1               5                   10                  15
His Leu Asp Ile Ile Trp,
                20

Cys Ser Cys Asn Ser Thr Leu Asp Lys Glu Cys Val Tyr Phe Cys
1               5                   10                  15
His Leu Asp Ile Ile Trp,
                20
 
H Ser Cys Phe Ser Ser Leu Asp Lys Glu Ala Val Tyr Phe Cys
1               5                   10                  15
His Leu Asp Ile Ile Trp,
                20

H Ser Cys Asn Ser Trp Leu Asp Lys Glu Ala Val Tyr Phe Cys
1               5                   10                  15
His Leu Asp Ile Ile Trp,
                20

Cys Ser Ala Asn Ser Trp Leu Asp Lys Glu Ala Val Tyr Phe Cys
 1              5                   10                  15
His Leu Asp Ile Ile Trp,
                20
 und ihre Derivate, die gegebenenfalls ein oder zwei Disulfid-Brücken aufweisen.

2. Peptid-Derivat mit dem folgenden Namen : 
Cys Ser Cys Phe Ser Ser Leu Asp Lys Glu Cys Val Tyr Phe Cys
1               5                   10                  15
His Leu Asp Ile Ile Trp,
                20
 und seine Derivate, die ein oder zwei Disulfid-Brücken aufweisen.

3. Verfahren zur Herstellung von neuen Peptid-Derivaten wie in Anspruch 1 definiert, dadurch gekennzeichnet, daß man eine Synthese in fester Phase durchführt, indem man sequentiell auf einen Träger vom Typ vernetzten Polystyrols die ordnungsgemäß geschützten Aminosäuren mit Hilfe eines Kopplungsmittels aufbringt, die Schutzgruppen der Aminosäuren entfernt und die auf diese Weise gebildete Peptidkette von dem Harz freisetzt und anschließend gegebenenfalls die Disulfid-Brücke(n) cyclisiert, um die gesuchten Peptid-Derivate zu erhalten.

4. Verfahren nach Anspruch 3, dadurch gekennzeichnet, daß :
- der Träger vom Typ vernetzten Polystyrols ein Harz des Typs "Boc-X₂₁-CM" ist, in dem Boc eine tert.-Butyloxycarbonyl-Gruppe ist, X₂₁ die bereits angegebene Bedeutung besitzt und CM den Träger aus vernetztem Polystyrol bezeichnet,
- das Kopplungsmittel (Benzotriazol-1-yloxy)-tris-(dimethylamino)-phosphonium-hexafluorphosphat oder Bop ist,
- die Freisetzung der Peptidkette von dem Träger sowie die Entfernung der Schutzgruppen von den Aminosäuren mit Hilfe von Fluorwasserstoffsäure durchgeführt wird, wobei man bei niedriger Temperatur, vorzugsweise bei etwa 0 °C arbeitet.

5. Medikamente, dadurch gekennzeichnet, daß sie aus den neuen Peptid-Derivaten, wie in Anspruch 1 definiert, bestehen.

6. Pharmazeutische Zusammensetzungen, dadurch gekennzeichnet, daß sie als Wirkstoff mindestens eines der wie in Anspruch 5 definierten Medikamente umfassen.


LISTE DE SEQUENCES

(1) INFORMATION GENERALE :

(i) DEPOSANT :

(A) NOM : ROUSSEL-UCLAF
(B) RUE : 35, Boulevard des Invalides
(C) VILLE : PARIS
(E) PAYS : FRANCE
(F) CODE POSTAL : 75007

(ii) TITRE DE L'INVENTION :

Nouveaux dérivés peptidiques, procédé de préparation et
application à titre de médicaments de ces nouveaux dérivés.

(iii) NOMBRE DE SEQUENCES : 17

(iv) FORME LISIBLE PAR ORDINATEUR :

(A) TYPE DE SUPPORT : Floppy Disk 5,25 pouces
(B) ORDINATEUR : IBM PC compatible
(C) SYSTEME D'EXPLOITATION : PC-DOS/MS-DOS
(D) LOGICIEL : Wordperfect 5.1

OEB

(v) DONNEES DE LA DEMANDE ACTUELLE :

NUMERO DE DEPOT : FR 92 01557

(2) INFORMATION POUR LA SEQ ID N° : 1

(i) CARACTERISTIQUES DE LA SEQUENCE :

(A) LONGUEUR : 21 acides aminés
(B) TYPE : peptide
(C) NOMBRE DE BRINS : simple
(D) CONFIGURATION : linéaire

(ii) TYPE DE MOLECULE : peptide

(xi) DESCRIPTION DE LA SEQUENCE SEQ ID N°1 :

Cys Ser Cys Asn Ser Ser Leu Asp Lys Glu Cys Val
1               5                   10
Tyr Phe Cys His Leu Asp Ile Ile H
15 20

(2) INFORMATION POUR LA SEQ ID N° : 2

(i) CARACTERISTIQUES DE LA SEQUENCE :

(A) LONGUEUR : 21 acides aminés
(B) TYPE : peptide
(C) NOMBRE DE BRINS : simple
(D) CONFIGURATION : linéaire

(ii) TYPE DE MOLECULE : peptide

(xi) DESCRIPTION DE LA SEQUENCE SEQ ID N° 2 :

Cys Ser Cys Asn Ser Trp Leu Asp Lys Glu Cys Val
1               5                   10
Tyr Phe Cys His Leu Asp Ile Ile H
15 20

(2) INFORMATION POUR LA SEQ ID N° : 3

(i) CARACTERISTIQUES DE LA SEQUENCE :

(A) LONGUEUR : 21 acides aminés
(B) TYPE : peptide
(C) NOMBRE DE BRINS : simple
(D) CONFIGURATION : linéaire

(ii) TYPE DE MOLECULE : peptide

(xi) DESCRIPTION DE LA SEQUENCE SEQ ID N° 3 :

Cys Ser Cys Phe Ser Trp Leu Asp Lys Glu Cys Val
1               5                   10

Tyr Phe Cys His Leu Asp Ile Ile Trp
        15                  20

(2) INFORMATION POUR LA SEQ ID N° : 4

(i) CARACTERISTIQUES DE LA SEQUENCE :

(A) LONGUEUR : 21 acides aminés
(B) TYPE : peptide
(C) NOMBRE DE BRINS : simple
(D) CONFIGURATION : linéaire

(ii) TYPE DE MOLECULE : peptide

(xi) DESCRIPTION DE LA SEQUENCE SEQ ID N° 4 :

Cys Ser Cys Phe Ser Ser Leu Asp Lys Glu Cys Val
1               5                   10
Tyr Phe Cys His Leu Asp Ile Ile Trp
        15                  20

(2) INFORMATION POUR LA SEQ ID N° : 5

(i) CARACTERISTIQUES DE LA SEQUENCE :

(A) LONGUEUR : 21 acides aminés
(B) TYPE : peptide
(C) NOMBRE DE BRINS : simple
(D) CONFIGURATION : linéaire

(ii) TYPE DE MOLECULE : peptide

(xi) DESCRIPTION DE LA SEQUENCE SEQ ID N° 5 :

Cys Ser Cys Asn Ser Tyr Leu Asp Lys Glu Cys Val
1               5                   10
Tyr Phe Cys His Leu Asp Ile Ile Trp
        15                  20

(2) INFORMATION POUR LA SEQ ID N° : 6

(i) CARACTERISTIQUES DE LA SEQUENCE :

(A) LONGUEUR : 21 acides aminés
(B) TYPE : peptide
(C) NOMBRE DE BRINS : simple
(D) CONFIGURATION : linéaire

(ii) TYPE DE MOLECULE : peptide

(xi) DESCRIPTION DE LA SEQUENCE SEQ ID N° 6 :

Cys Ser Cys Phe Ser Ser Leu Asp Glu Glu Cys Val
1               5                   10
Tyr Phe Cys His Leu Asp Ile Ile Trp
        15                  20

(2) INFORMATION POUR LA SEQ ID N° : 7

(i) CARACTERISTIQUES DE LA SEQUENCE :

(A) LONGUEUR : 21 acides aminés
(B) TYPE : peptide
(C) NOMBRE DE BRINS : simple
(D) CONFIGURATION : linéaire

(ii) TYPE DE MOLECULE : peptide

(xi) DESCRIPTION DE LA SEQUENCE SEQ ID N° 7 :

Cys Ser Cys Gly Ser Trp Leu Asp Lys Glu Cys Val
1               5                   10
Tyr Phe Cys His Leu Asp Ile Ile Trp
        15                  20

(2) INFORMATION POUR LA SEQ ID N° : 8

(i) CARACTERISTIQUES DE LA SEQUENCE :

(A) LONGUEUR : 21 acides aminés
(B) TYPE : peptide
(C) NOMBRE DE BRINS : simple
(D) CONFIGURATION : linéaire

(ii) TYPE DE MOLECULE : peptide

(xi) DESCRIPTION DE LA SEQUENCE SEQ ID N° 8 :

Cys Ser Cys Asn Ser Ser Leu Asp Lys Glu Cys Val
1               5                   10
Tyr Phe Cys His Leu Asp Ile Ile Trp
        15                  20

(2) INFORMATION POUR LA SEQ ID N° : 9

(i) CARACTERISTIQUES DE LA SEQUENCE :

(A) LONGUEUR : 21 acides aminés
(B) TYPE : peptide
(C) NOMBRE DE BRINS : simple
(D) CONFIGURATION : linéaire

(ii) TYPE DE MOLECULE : peptide

(xi) DESCRIPTION DE LA SEQUENCE SEQ ID N° 9 :

Cys Ser Cys Phe Ser Asp Leu Asp Lys Glu Cys Val
1               5                   10
Tyr Phe Cys His Leu Asp Ile Ile Trp
        15                  20

(2) INFORMATION POUR LA SEQ ID N° : 10

(i) CARACTERISTIQUES DE LA SEQUENCE :

(A) LONGUEUR : 21 acides aminés
(B) TYPE : peptide
(C) NOMBRE DE BRINS : simple
(D) CONFIGURATION : linéaire

(ii) TYPE DE MOLECULE : peptide

(xi) DESCRIPTION DE LA SEQUENCE SEQ ID N° 10 :

Cys Ser Cys Phe Ser Lys Leu Asp Lys Glu Cys Val
1               5                   10
Tyr Phe Cys His Leu Asp Ile Ile Trp
        15                  20

(2) INFORMATION POUR LA SEQ ID N° : 11

(i) CARACTERISTIQUES DE LA SEQUENCE :

(A) LONGUEUR : 21 acides aminés
(B) TYPE : peptide
(C) NOMBRE DE BRINS : simple
(D) CONFIGURATION : linéaire

(ii) TYPE DE MOLECULE : peptide

(xi) DESCRIPTION DE LA SEQUENCE SEQ ID N° 11 :

Cys Ser Cys Phe Ser Thr Leu Asp Lys Glu Cys Val
1               5                   10
Tyr Tyr Cys His Leu Asp Ile Ile Trp
        15                  20

(2) INFORMATION POUR LA SEQ ID N° : 12

(i) CARACTERISTIQUES DE LA SEQUENCE :

(A) LONGUEUR : 21 acides aminés
(B) TYPE : peptide
(C) NOMBRE DE BRINS : simple
(D) CONFIGURATION : linéaire

(ii) TYPE DE MOLECULE : peptide

(xi) DESCRIPTION DE LA SEQUENCE SEQ ID N° 12 :

Cys Ser Cys Asn Ser Trp Leu Asp Lys Glu Cys Val
 1               5                   10
Tyr Phe Cys His Asp Asp Ile Ile Trp
        15                  20

(2) INFORMATION POUR LA SEQ ID N° : 13

(i) CARACTERISTIQUES DE LA SEQUENCE :

(A) LONGUEUR : 21 acides aminés
(B) TYPE : peptide
(C) NOMBRE DE BRINS : simple
(D) CONFIGURATION : linéaire

(ii) TYPE DE MOLECULE : peptide

(xi) DESCRIPTION DE LA SEQUENCE SEQ ID N° 13 :

Cys Ser Ala Phe Ser Ser Leu Asp Lys Glu Ala Val
1               5                   10
Tyr Phe Cys His Leu Asp Ile Ile Trp
        15                  20

(2) INFORMATION POUR LA SEQ ID N° : 14

(i) CARACTERISTIQUES DE LA SEQUENCE :

(A) LONGUEUR : 21 acides aminés
(B) TYPE : peptide
(C) NOMBRE DE BRINS : simple
(D) CONFIGURATION : linéaire

(ii) TYPE DE MOLECULE : peptide

(xi) DESCRIPTION DE LA SEQUENCE SEQ ID N° 14 :

Cys Ser Cys Asn Ser Thr Leu Asp Lys Glu Cys Val
1               5                   10
Tyr Phe Cys His Leu Asp Ile Ile Trp
        15                  20

(2) INFORMATION POUR LA SEQ ID N° : 15

(i) CARACTERISTIQUES DE LA SEQUENCE :

(A) LONGUEUR : 21 acides aminés
(B) TYPE : peptide
(C) NOMBRE DE BRINS : simple
(D) CONFIGURATION : linéaire

(ii) TYPE DE MOLECULE : peptide

(xi) DESCRIPTION DE LA SEQUENCE SEQ ID N° 15 :

H Ser Cys Phe Ser Ser Leu Asp Lys Glu Ala Val
1 5 10
Tyr Phe Cys His Leu Asp Ile Ile Trp
15 20

(2) INFORMATION POUR LA SEQ ID N° : 16

(i) CARACTERISTIQUES DE LA SEQUENCE :

(A) LONGUEUR : 21 acides aminés
(B) TYPE : peptide
(C) NOMBRE DE BRINS : simple
(D) CONFIGURATION : linéaire

(ii) TYPE DE MOLECULE : peptide

(xi) DESCRIPTION DE LA SEQUENCE SEQ ID N° 16 :

H Ser Cys Asn Ser Trp Leu Asp Lys Glu Ala Val
1 5 10
Tyr Phe Cys His Leu Asp Ile Ile Trp
15 20

(2) INFORMATION POUR LA SEQ ID N° : 17

(i) CARACTERISTIQUES DE LA SEQUENCE :

(A) LONGUEUR : 21 acides aminés
(B) TYPE : peptide
(C) NOMBRE DE BRINS : simple
(D) CONFIGURATION : linéaire

(ii) TYPE DE MOLECULE : peptide

(xi) DESCRIPTION DE LA SEQUENCE SEQ ID N° 17 :

Cys Ser Ala Asn Ser Trp Leu Asp Lys Glu Ala Val
1               5                   10
Tyr Phe Cys His Leu Asp Ile Ile Trp
        15                  20